(19) Europäisches Patentamt
European Patent Office
Office européen des brevets

(11) Numéro de publication : **0 449 693 B1**

(12) # FASCICULE DE BREVET EUROPEEN

(45) Date de publication du fascicule du brevet : **01.06.94 Bulletin 94/22**

(51) Int. Cl.$^5$ : **C07C 69/24,** C07C 67/38

(21) Numéro de dépôt : **91400683.8**

(22) Date de dépôt : **13.03.91**

(54) **Catalyseur et procédé pour la synthèse d'esters carboxyliques saturés.**

(30) Priorité : **26.03.90 FR 9003805**

(43) Date de publication de la demande : **02.10.91 Bulletin 91/40**

(45) Mention de la délivrance du brevet : **01.06.94 Bulletin 94/22**

(84) Etats contractants désignés : **AT BE CH DE DK ES FR GB GR IT LI LU NL SE**

(56) Documents cités :
**EP-A- 0 219 948**
**EP-A- 0 310 168**
**JP-A-63 077 846**

(73) Titulaire : **ELF ATOCHEM S.A.**
**4 & 8, Cours Michelet**
**La Défense 10**
**F-92800 Puteaux (FR)**

(72) Inventeur : **Legrand, Christophe**
**Résidence America 2B,**
**Appt. 63**
**F-59370 Mons-en-Baroeul (FR)**
Inventeur : **Castanet, Yves**
**11 Allée P. Bonard**
**F-59510 Hem (FR)**
Inventeur : **Mortreux, André**
**17 Reu Léon Gambetta**
**F-59510 Hem (FR)**
Inventeur : **Petit, Francis**
**13 Allée de la Clairière**
**F-59650 Villeneuve D'Ascq (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention se rapporte à une famille de catalyseurs et à un procédé pour la synthèse d'esters carboxyliques saturés à partir de formiate d'alkyle et d'une oléfine.

Il est connu de préparer des esters carboxyliques saturés par réaction de formiate d'alkyle et d'une oléfine sous pression, à température relativement élevée et en présence d'un système catalytique comprenant un dérivé de métal de transition. Par exemple le brevet EP-A-106 656 décrit la réaction du formiate de méthyle avec l'éthylène dans l'acide acétique comme solvant, à 200°C et sous une pression de 25 bars, en présence d'un système catalytique comprenant du trichlorure d'iridium, de l'iodure de méthyle en tant que promoteur et de l'acide paratoluènesulfonique en tant que copromoteur ; après 30 minutes de réaction, le mélange réactionnel contient d'une part 10,5% de formiate de méthyle non converti et d'autre part un mélange de produits contenant 12,7% de propionate de méthyle, ce qui permet d'évaluer à 330 $h^{-1}$ environ l'activité du catalyseur en propionate de méthyle, définie comme le nombre de moles de formiate de méthyle transformées en propionate de méthyle par mole de catalyseur et par heure. Dans ce procédé non seulement la sélectivité en propionate de méthyle est très faible mais encore la rareté et le prix de l'iridium rendent la synthèse du propionate de méthyle peu économique. La publication de W.KEIM et J.BECKER dans Journal of Molecular Catalysis, 54 (1989) 95-101 décrit la même réaction effectuée pendant 20 heures à 230°C sous 90 bars dans le toluène en présence de $Ru_3(CO)_{12}$ comme catalyseur : dans ces conditions la conversion est de 100% et la sélectivité en propionate de méthyle de 92%, ce qui permet d'évaluer à 230 $h^{-1}$ l'activité du catalyseur en propionate de méthyle, telle que définie précédemment. Cette technique constitue un progrès par rapport à celle du brevet EP-A-106 656 dans la mesure où une excellente sélectivité peut être obtenue au moyen d'un catalyseur de ruthénium plus accessible commercialement que celui d'iridium. Cependant l'activité du catalyseur en propionate de méthyle reste modérée et même inférieure à celle du brevet déjà cité.

HIDAI et al. ont publié dans Journal of Molecular Catalysis, 40 (1987) 243-254 la réaction, effectuée en présence de divers systèmes catalytiques à base de ruthénium et d'iodures, de l'éthylène avec un mélange équimolaire de monoxyde de carbone et de méthanol. La publication montre qu'à 190°C et en présence de $Ru_3(CO)_{12}$ comme catalyseur, les meilleurs résultats sont obtenus en utilisant l'iodure de sodium ou de lithium comme promoteur, de préférence à l'iodure de phényle, l'iodure de tétra-n-butylamine ou l'iodure de tétraphénylphosphine. La sélectivité est bonne et l'activité catalytique en propionate de méthyle augmente avec le rapport molaire de l'iodure de sodium au ruthénium, pouvant atteindre jusqu'à 1220 $h^{-1}$ lorsque le rapport I/Ru est égal à 10. Ce procédé présente toutefois deux graves inconvénients : d'une part la réaction nécessite le recours à du monoxyde de carbone gazeux dont la manipulation et le transport sont dangereux, ce qui a pour effet de limiter la production de propionate de méthyle à proximité de sites industriels producteurs de ce gaz ; d'autre part l'emploi de quantités importantes d'iodures est une cause bien connue de corrosion des réacteurs en acier dans lesquels la réaction serait mise en oeuvre.

Un premier problème que la présente invention vise à résoudre consiste donc à mettre au point un catalyseur et un procédé capables de produire le propionate de méthyle avec une sélectivité élevée et une activité catalytique élevée à partir d'une matière première évitant le recours au monoxyde de carbone. Un second problème que vise à résoudre la présente invention consiste à mettre au point un catalyseur permettant de produire le propionate de méthyle avec une sélectivité élevée et une activité élevée sans avoir recours à des quantités importantes d'iodures, de manière à limiter les risques de corrosion des réacteurs.

La présente invention est basée sur la découverte que ces deux problèmes peuvent être simultanément résolus en effectuant la synthèse du propionate de méthyle à partir de formiate de méthyle et d'éthylène, sans présence substantielle de monoxyde de carbone, à une température de 120° à 280°C environ - de préférence 160° à 250°C -, sous une pression de 1 à 3000 bars - de préférence 20 à 200 bars -, en présence d'un catalyseur à base de ruthénium coordiné par des ligands choisis parmi le monoxyde de carbone, les atomes d'halogène et les amines, et en présence d'un amide comme solvant. En outre il a été découvert que ces conditions réactionnelles peuvent être appliquées avec succès à la synthèse d'esters carboxyliques saturés à partir de formiate d'alkyle et d'oléfine selon la réaction :

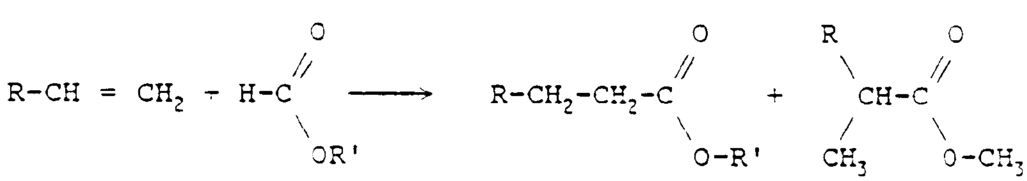

dans laquelle R est choisi parmi l'atome d'hydrogène et les radicaux alkyle ayant, de préférence, 1 à 8 atomes

de carbone, et R′ désigne un radical alkyle possédant de préférence 1 à 8 atomes de carbone. Dans le cas du formiate de méthyle et de l'éthylène, le procédé selon l'invention permet d'obtenir le propionate de méthyle avec une activité très élevée et, en raison de la forte sélectivité, permet de l'isoler très aisément du milieu réactionnel.

Le catalyseur utilisé dans le procédé selon l'invention est à base de ruthénium coordiné par des atomes d'halogène et/ou des molécules de monoxyde de carbone ou d'amines telles que l'ammoniac, la propylamine, la triéthylamine, la pipéridine, ou l'éthylènediamine. Il forme un complexe qui peut être mono-, bi- ou trimétallique. De préférence un tel catalyseur comprend au moins un atome d'halogène comme ligand. Des exemples de tels catalyseurs comprennent par exemple le trichlorure de ruthénium $RuCl_3,3H_2O$, l'iodure de ruthénium $RuI_3$, le bis(dichlorotricarbonylruthénium) $[RuCl_2(CO)_3]_2$ et le tris(tétracarbonylruthénium) $Ru_3(CO)_{12}$ ainsi que les composés $Ru(NH_3)_6Cl_3$ et $[Ru(NH_3)_5Cl]Cl_2$. Le cas échéant le catalyseur peut comprendre des ligands autres qu'un atome d'halogène ou le monoxyde de carbone, à l'exception toutefois des phosphines dont la présence en quantité substantielle est déconseillée car elle diminue fortement l'activité catalytique, sauf en présence d'iode en quantité excédentaire par rapport à la phosphine.

Le procédé selon l'invention peut être mis en oeuvre en la présence supplémentaire d'iode, d'un iodure covalent ou d'un halogénure d'ammonium quaternaire jouant le rôle pas nécessairement de promoteur de l'activité catalytique mais de stabilisant de l'espèce catalytique active. Comme exemples d'iodures covalents on peut citer les iodures de méthyle, d'hydrogène, d'éthyle, de n-butyle et de phényle. Comme exemples d'halogénures d'ammonium quaternaires on peut citer l'iodure de tétraéthylammonium et le chlorure de tétra-n-butylammonium. Le rapport molaire de l'iode, l'iodure covalent ou l'halogénure d'ammonium quaternaire, relativement au catalyseur à base de ruthénium est de préférence compris entre 0,5 et 6 environ, plus particulièrement entre 1 et 2,5. L'addition d'halogénure de phosphonium quaternaire est déconseillée car elle diminue l'activité catalytique.

Le rapport molaire du formiate d'alkyle au ruthénium dans le procédé selon l'invention est de préférence compris entre 500 et 10000 environ. Le formiate d'alkyle et l'oléfine sont d'autre part dans un rapport sensiblement équimolaire. La réaction selon l'invention est effectuée en solution dans un amide car le recours à d'autres solvants (toluène, acétone, tétrahydrofuranne) s'est révélé inefficace. Comme exemples d'amides pouvant être utilisés on peut citer notamment la N-méthylpyrrolidone, le diméthylacétamide, le formamide, le méthylformamide et le diméthylformamide, celui-ci étant préféré. La quantité de solvant peut être choisie dans une large gamme, étant entendu qu'à performances équivalentes on tendra à utiliser la quantité de solvant la plus faible possible de manière à simplifier les problèmes de séparation de l'ester carboxylique saturé formé à l'issue de la réaction. En règle générale, on utilise au moins 0,5 volume, de préférence 1 à 2 volumes, et au plus 3 volumes environ d'amide pour 1 volume de formiate d'alkyle (étant rappelé par exemple que sous pression atmosphérique le formiate de méthyle est un liquide de température d'ébullition égale à 33°C).

Il est important que la réaction selon l'invention soit effectuée sans présence substantielle de monoxyde de carbone car on a observé que l'addition de monoxyde de carbone au mélange réactionnel a pour résultat de diminuer l'activité catalytique. En général on pourra tolérer la présence de jusqu'à 10% environ en volume de monoxyde de carbone par rapport à l'éthylène, de préférence jusqu'à 5% en volume environ. Des proportions de monoxyde de carbone supérieures à ces valeurs seront considérées, selon la présente invention, comme une présence substantielle de monoxyde de carbone.

La réaction selon l'invention est effectuée, de préférence, sans présence substantielle d'eau car la présence d'eau a pour résultat de diminuer l'activité catalytique. En général, on pourra tolérer la présence d'eau dans un rapport molaire allant jusqu'à 20 environ par rapport au ruthénium.

La pression et la température réactionnelles, choisies dans les gammes indiquées ci-dessus, sont naturellement dépendantes l'une de l'autre et pourront être déterminées par l'homme de l'art en fonction de la technologie retenue (type de réacteur, moyens de compression, etc.) en vue d'optimiser l'efficacité de la réaction, notamment l'activité catalytique, pour chaque catalyseur.

La durée de la réaction est naturellement fonction de la pression et de la température choisies et sera d'autant plus longue que celles-ci seront plus basses. Dans les gammes de pression et de température indiquées ci-dessus, une durée réactionnelle suffisante pour convertir la quasi-totalité du formiate d'alkyle est généralement comprise entre 2 minutes et 10 heures.

Lorsque l'oléfine utilisée dans le procédé selon l'invention comprend plus de 2 atomes de carbone (R est un radical alkyle ayant de 1 à 8 atomes de carbone), la réaction conduit à la formation d'un mélange d'isomères linéaire et ramifié de l'ester carboxylique saturé. En général la réaction privilégie la formation de l'un ces isomères dans le mélange et il est à la portée de l'homme de l'art de choisir l'un des systèmes catalytiques décrits précédemment en vue de préparer préférentiellement l'un de ces isomères.

La technologie nécessaire à la mise en oeuvre industrielle de ce type de réaction est bien connue de l'homme de l'art. En fin de réaction, le mélange réactionnel est généralement évacué du réacteur et soumis à une

distillation éclair en vue d'éliminer le mélange d'ester(s) carboxylique(s) saturé(s) et de formiate d'alkyle non converti, à partir duquel les produits désirés sont séparés dans une colonne de distillation, tandis que l'amide et le catalyseur sont recyclés vers le réacteur.

Parmi les catalyseurs proposés par la présente invention pour la synthèse d'esters carboxyliques satures à partir de formiate d'alkyle et d'une oléfine, certains n'avaient jamais été proposés pour cette réaction. Il en va ainsi :

- d'un catalyseur constitué essentiellement de trichlorure de ruthénium $RuCl_3$, $3H_2O$, c'est-à-dire ne comprenant pas d'autre composant à l'exclusion d'éventuelles impuretés ;
- d'un catalyseur constitué essentiellement de la combinaison d'un trichlorure de ruthénium $RuCl_3$, $3H_2O$ associé à au moins un stabilisant choisi parmi l'iode, les iodures covalents et les halogénures d'ammonium quaternaires, à l'exclusion de tout composant autre que d'éventuelles impuretés ;
- d'un catalyseur comprenant du tris(tétracarbonylruthénium) $Ru_3(CO)_{12}$ associé à au moins un stabilisant choisi parmi l'iode, les iodures covalents et les halogénures d'ammonium quaternaires et pouvant éventuellement comprendre d'autres composants catalytiques ;
- d'un catalyseur choisi parmi l'iodure de ruthénium $RuI_3$, le bis(dichlorotricarbonylruthénium) $[RuCl_2(CO)_3]_2$, et les composés $Ru(NH_3)_6Cl_3$ et $[Ru(NH_3)_5Cl]Cl_2$, comprenant le cas échéant en outre au moins un stabilisant choisi parmi l'iode, les iodures covalents et les halogénures d'ammonium quaternaires et le cas échéant d'autres composants catalytiques.

La présente invention permet une préparation simple et efficace d'esters carboxyliques saturés tels que le propionate de méthyle (à partir ce l'éthylène), le butyrate et l'isobutyrate de méthyle (à partir du propène), ce dernier ayant une importance particulière en raison de la possibilité de le déshydrogéner en méthacrylate de méthyle.

Les exemples suivants sont donnés à titre illustratif et non limitatif de la présente invention.

## EXEMPLE 1

Dans un tube de Schlenk on mélange sous azote 0,11 millimole de trichlorure de ruthénium $RuCl_3$, $3H_2O$ et 0,25 millimole d'iodure de tétraéthylammonium dans 30 $cm^3$ de diméthylformamide dégazé. On transfère ce système catalytique dans un réacteur autoclave de 100 $cm^3$ muni d'une agitation mécanique et préalablement purgé par de l'azote. Dans ce réacteur on introduit de l'éthylène jusqu'à une pression de 55 bars à 20°C puis, en maintenant l'agitation, on chauffe jusqu'à 190°C et quand cette température est atteinte on introduit 0,26 mole de formiate de méthyle (16 $cm^3$) à l'aide d'une pompe à injection sous pression. Après une heure de réaction, on laisse le réacteur refroidir et on récupère le mélange réactionnel qui est analysé par chromatographie en phase gazeuse. Dans le tableau ci-après sont indiqués le taux de conversion C du formiate de méthyle ainsi que la sélectivité S en propionate de méthyle dans les produits formés et l'activité A exprimée en $h^{-1}$, définie comme le nombre de moles de formiate de méthyle transformées en propionate de méthyle par mole de catalyseur (Ru) et par heure.

## EXEMPLE 2

La procédure expérimentale de l'Exemple 1 est reproduite en modifiant la température : 180°C au lieu de 190°C. Les résultats sont indiqués au tableau ci-après.

## EXEMPLE 3 à 8

La procédure expérimentale de l'Exemple 1 est reproduite en remplaçant l'iodure de tétraéthylammonium par un autre composé d'iode :

- iodure de phényle (Exemple 3),
- iodure d'hydrogène (Exemple 4),
- iode (Exemple 5), la durée de la réaction étant portée à 2 heures dans ce cas,
- iodure de méthyle (Exemple 6),
- iodure d'éthyle (Exemple 7),
- 1-iodobutane (Exemple 8).

Les résultats sont indiqués au tableau ci-après.

## EXEMPLE 9 (Comparatif)

La procédure expérimentale de l'Exemple 6 est reproduite en ajoutant au milieu réactionnel 0,11 millimole

de triphénylphosphine susceptible de jouer le rôle de ligand pour le ruthénium. Les résultats sont indiqués au tableau ci-après.

EXEMPLE 10

La procédure expérimentale de l'Exemple 5 est reproduite en ajoutant au milieu réactionnel 0,25 millimole de triphénylphosphine susceptible de jouer le rôle de ligand pour le ruthénium. Les résultats sont indiqués au tableau ci-après.

EXEMPLE 11 (Comparatif)

On reproduit le mode opératoire de l'Exemple 10, à l'exception de la quantité de triphénylphosphine qui est portée à 1 millimole. Les résultats sont indiqués au tableau ci-après.

EXEMPLE 12 (Comparatif)

La procédure expérimentale de l'Exemple 1 est reproduite en remplaçant l'iodure de triéthylammonium par l'iodure de méthyltriphénylphosphonium. Les résultats sont indiqués au tableau ci-après.

EXEMPLE 13

La procédure expérimentale de l'Exemple 1 est reproduite en ajoutant une pression de 3 bars de monoxyde de carbone dans le réacteur. Les résultats sont indiqués au tableau ci-après.

EXEMPLE 14 (Comparatif)

On reproduit le mode opératoire de l'Exemple 13, à l'exception de la pression de monoxyde de carbone dans le réacteur qui est portée à 30 bars tandis que la pression d'éthylène est abaissée à 30 bars. Les résultats sont indiqués au tableau ci-après.

EXEMPLE 15

La procédure expérimentale de l'Exemple 1 est reproduite aux exceptions suivantes près :

- l'éthylène est remplacée par le propène,
- la pression dans le réacteur est portée à 100 bars,
- la température dans le réacteur est abaissée à 160°C.

Après 6 heures de réaction, on obtient avec un rendement de 31% un mélange de butyrates de méthyle dans lequel le rapport molaire $\frac{\text{ramifié}}{\text{normal}}$ est égal à 1,22.

EXEMPLE 16

On reproduit la procédure expérimentale de l'Exemple 6 en remplaçant le trichlorure de ruthénium par le bis(dichlorotricarbonylruthénium) $[RuCl_2(CO)_3]_2$. Les résultats sont indiqués au tableau ci-après.

EXEMPLE 17

On reproduit la procédure expérimentale de l'Exemple 16 en remplaçant l'iodure de méthyle par l'iodure de tétraéthylammonium. Les résultats sont indiqués au tableau ci-après.

EXEMPLE 18

On reproduit la procédure expérimentale de l'Exemple 1 en remplaçant le trichlorure de ruthénium par le tris(tétracarbonylruthénium) $Ru_3(CO)_{12}$. Les résultats sont indiqués au tableau ci-après.

EXEMPLE 19

On reproduit la procédure expérimentale de l'Exemple 1 en remplaçant le trichlorure de ruthénium par le

triiodure de ruthénium et l'iodure de tétraéthylammonium par le chlorure de tétra-n-butylammonium. Les résultats sont indiqués au tableau ci-après.

EXEMPLES 20 et 21

On reproduit la procédure expérimentale de l'Exemple 4 en diminuant la quantité d'iodure d'hydrogène, qui est portée respectivement à 0,06 millimole (Exemple 20) et 0,13 millimole (Exemple 21). Les résultats sont indiqués au tableau ci-après.

EXEMPLE 22

On reproduit la procédure expérimentale de l'Exemple 1 en ajoutant dans le réacteur, dès le début, 0,5 millimole d'eau. Les résultats sont indiqués au tableau ci-après.

EXEMPLE 23

On reproduit la procédure expérimentale de l'Exemple 2 aux exceptions suivantes près :
- le formiate de méthyle est introduit dans le réacteur à froid avant l'éthylène,
- la réaction est poursuivie pendant 2 heures.

Les résultats sont indiqués au tableau ci-après.

EXEMPLE 24 (comparatif)

On reproduit la procédure expérimentale de l'Exemple 5 aux deux exceptions suivantes près :
- le diméthylformamide est remplacé par le toluène,
- la durée de réaction est portée à 10 heures.

A l'issue de ce temps de réaction, l'analyse du milieu ne révèle la présence d'aucune trace de propionate de méthyle.

EXEMPLE 25

On reproduit la procédure expérimentale de l'Exemple 1 en introduisant dans le réacteur, en même temps que le formiate de méthyle, 1 $cm^3$ de méthanol deuteré $CD_3OD$. Le résultat obtenu est identique à celui de l'exemple 1 et l'analyse du milieu ne révèle la présence d'aucune trace de propionate de méthyle deuteré, ce qui prouve que le méthanol ne joue aucun rôle dans le mécanisme de formation du propionate à partir du formiate de méthyle.

EXEMPLE 26

On reproduit la procédure expérimentale de l'Exemple 1 aux deux exceptions suivantes près :
- le trichlorure de ruthénium est remplacé par le composé $Ru(NH_3)_6Cl_3$ ;
- la température réactionnelle est abaissée à 170°C.

Les résultats sont indiqués au tableau ci-après.

EXEMPLE 27

On reproduit la procédure expérimentale de l'Exemple 1 en remplaçant le trichlorure de ruthénium par le composé $[Ru(NH_3)_5Cl]Cl_2$.

Les résultats sont indiqués au tableau ci-après.

TABLEAU

| Exemple | C (%) | S (%) | A ($h^{-1}$) |
|---|---|---|---|
| 1 | 77 | 84 | 1530 |
| 2 | 43 | 91 | 920 |
| 3 | 77 | 84 | 1530 |
| 4 | 66 | 92 | 1440 |
| 5 | 61 | 88 | 635 |
| 6 | 65 | 94 | 1440 |
| 7 | 65,5 | 93 | 1440 |
| 8 | 71 | 94 | 1580 |
| 9 | 35 | 59 | 490 |
| 10 | 50 | 85 | 1000 |
| 11 | 6 | 46 | 65 |
| 12 | 71 | 14 | 235 |
| 13 | 36 | 87 | 730 |
| 14 | 45 | 11 | 120 |
| 16 | 52 | 87 | 1060 |
| 17 | 56 | 96 | 1490 |
| 18 | 34,5 | 97 | 790 |
| 19 | 50,5 | 92 | 1100 |
| 20 | 56 | 78 | 1030 |
| 21 | 66 | 87 | 1360 |
| 22 | 65 | 67 | 1030 |
| 23 | 36 | 97 | 980 |
| 26 | 31 | 91 | 670 |
| 27 | 35 | 96 | 800 |

## Revendications

1. Procédé de synthèse d'esters carboxyliques saturés à partir de formiate d'alkyle et d'une oléfine, sans présence substantielle de monoxyde de carbone, à une température de 120° à 280°C, sous une pression de 1 à 3000 bars, en présence d'un catalyseur à base de ruthénium coordiné par des ligands choisis parmi le monoxyde de carbone, les atomes d'halogène et les amines, et en présence d'un amide comme solvant.

2. Procédé selon la revendication 1 pour la synthèse de propionate de méthyle, caractérisé en ce que le

formiate d'alkyle est le formiate de méthyle et en ce que l'oléfine est l'éthylène.

3. Procédé selon l'une des revendications 1 et 2, caractérisé en ce que le rapport molaire de formiate d'alkyle au ruthénium est compris entre 500 et 10 000.

4. Procédé selon l'une des revendications 1 à 3, caractérisé en ce qu'il est mis en oeuvre en la présence supplémentaire d'iode, d'un iodure covalent ou d'un halogénure d'ammonium quaternaire.

5. Procédé selon la revendication 4, caractérisé en ce que le rapport molaire de l'iode ou dudit iodure covalent ou halogénure d'ammonium quaternaire relativement au ruthénium est compris entre 0,5 et 6.

6. Procédé selon l'une des revendications 1 à 5, caractérisé en ce que le solvant est le diméthylformamide.

7. Procédé selon l'une des revendications 1 à 6, caractérisé en ce que l'amide est utilisé à raison de 0,5 à 3 volumes pour 1 volume de formiate d'alkyle.

8. Procédé selon l'une des revendications 1 à 7, caractérisé en ce que la réaction entre le formiate d'alkyle et l'oléfine est effectuée pendant une durée comprise entre 2 minutes et 10 heures.

9. Procédé selon l'une des revendications 1 à 8, caractérisé en ce que le formiate d'alkyle et l'oléfine sont dans un rapport sensiblement équimolaire.

10. Procédé selon l'une des revendications 1 à 9, caractérisé en ce que le catalyseur est choisi parmi :
   - les catalyseurs constitués essentiellement de trichlorure de ruthénium $RuCl_3,3H_2O$ ;
   - les catalyseurs constitués essentiellement de la combinaison d'un trichlorure de ruthénium $RuCl_3,3H_2O$ associé à au moins un stabilisant choisi parmi l'iode, les iodures covalents et les halogénures d'ammonium quaternaires
   - les catalyseurs comprenant du tris(tétracarbonylruthénium) $Ru_3(CO)_{12}$ associé à au moins un stabilisant choisi parmi l'iode, les iodures covalents et les halogénures d'ammonium quaternaires ; et
   - les catalyseurs choisis parmi l'iodure de ruthénium $RuI_3$, le bis(dichlorotricarbonylruthénium) $[RuCl_2(CO)_3]_2$ et les composés $Ru(NH_3)_6Cl_3$ et $[Ru(NH_3)_5Cl]Cl_2$, comprenant le cas échéant en outre au moins un stabilisant choisi parmi l'iode, les iodures covalents et les halogénures d'ammonium quaternaires.

11. Utilisation comme catalyseur pour la synthèse d'esters carboxyliques saturés à partir de formiate d'alkyle et d'une oléfine, d'au moins l'un parmi :
   - le trichlorure de ruthénium $RuCl_3,3H_2O$ ;
   - le trichlorure de ruthénium $RuCl_3,3H_2O$ associé à au moins un stabilisant choisi parmi l'iode, les iodures covalents et les halogénures d'ammonium quaternaires ;
   - le tris(tétracarbonylruthénium) $Ru_3(CO)_{12}$ associé à au moins un stabilisant choisi parmi l'iode, les iodures covalents et les halogénures d'ammonium quaternaires ; et
   - l'iodure de ruthénium $RuI_3$, le bis(dichlorotricarbonylruthénium) $[RuCl_2(CO)_3]_2$ et les composés $Ru(NH_3)_6Cl_3$ et $[Ru(NH_3)_5Cl]Cl_2$, comprenant le cas échéant en outre au moins un stabilisant choisi parmi l'iode, les iodures covalents et les halogénures d'ammonium quaternaires.

**Patentansprüche**

1. Verfahren zur Synthese von gesättigten Carbonsäureestern aus Alkylformiat und einem Olefin ohne Anwesenheit wesentlicher Mengen von Kohlenmonoxid, bei einer Temperatur von 120° bis 280°C, unter einem Druck von 1 bis 3000 bar, in Gegenwart eines Katalysators auf Rutheniumbasis, koordiniert durch Liganden, die ausgewählt sind aus der Gruppe bestehend aus Kohlenmonoxid, den Halogenatomen und den Aminen, und in Gegenwart eines Amids als Lösungsmittel.

2. Verfahren nach Anspruch 1, zur Synthese von Methylpropionat, dadurch gekennzeichnet, daß das Alkylformiat Methylformiat ist und das Olefin Ethylen ist.

3. Verfahren nach Anspruch 1 oder 2, dadurch gekennzeichnet, daß das Molverhältnis des Alkylformiats zum Ruthenium zwischen 500 und 10 000 beträgt.

4. Verfahren nach einem der Ansprüche 1 bis 3, dadurch gekennzeichnet, daß es in der zusätzlichen Gegenwart von Jod, einem kovalenten Jodid oder einem quartären Ammoniumhalogenid durchgeführtwird.

5. Verfahren nach Anspruch 4, dadurch gekennzeichnet, daß das Molverhältnis des Jods oder kovalenten Iodids oder quartären Ammoniumhalogenids zum Ruthenium zwischen 0,5 und 6 beträgt.

6. Verfahren nach einem der Ansprüche 1 bis 5, dadurch gekennzeichnet, daß das Lösungsmittel Dimethylformamid ist.

7. Verfahren nach einem der Ansprüche 1 bis 6, dadurch gekennzeichnet, daß das Amid in einer Menge von 0,5 bis 3 Volumen pro Volumen Alkylformiat verwendet wird.

8. Verfahren nach einem der Ansprüche 1 bis 7, dadurch gekennzeichnet, daß die Reaktion zwischen dem Alkylformiat und dem Olefin für eine Dauer zwischen 2 Minuten und 10 Stunden durchgeführt wird.

9. Verfahren nach einem der Ansprüche 1 bis 8, dadurch gekennzeichnet, daß das Alkylformiat und das Olefin in einem im wesentlichen äquimolaren Verhältnis stehen.

10. Verfahren nach einem der Ansprüche 1 bis 9, dadurch gekennzeichnet, daß der Katalysator ausgewählt wird aus:
- den Katalysatoren, die hauptsächlich aus Rutheniumtrichlorid $RuCl_3.3H_2O$ bestehen;
- den Katalysatoren, die hauptsächlich aus der Kombination eines Rutheniumtrichlorids $RuCl_3.3H_2O$ mit mindestens einem Stabilisator, ausgewählt aus der Gruppe bestehend aus Jod, den kovalenten Jodiden und den quartären Ammoniumhalogeniden, bestehen;
- den Katalysatoren, die tris-(Tetracarbonylruthenium) $Ru_3(CO)_{12}$ in Verbindung mit mindestens einem Stabilisator, ausgewählt aus der Gruppe bestehend aus Jod, den kovalenten Jodiden und den quartären Ammoniumhalogeniden, enthalten; und
- den Katalysatoren, die ausgewählt sind aus der Gruppe bestehend aus Rutheniumiodid $RuJ_3$, bis-(Dichlortricarbonylruthenium) $[RuCl_2(CO)_3]_2$ und den Verbindungen $Ru(NH_3)_6Cl_3$ und $[Ru(NH_3)_5Cl]Cl_2$ und gegebenenfalls weiters mindestens einen Stabilisator, ausgewählt aus der Gruppe bestehend aus Jod, den kovalenten Jodiden und den quartären Ammoniumhalogeniden, enthalten.

11. Verwendung mindestens eines der folgenden Katalysatoren als Katalysator für die Synthese von gesättigten Carbonsäureestern aus Alkylformiat und einem Olefin:
- Rutheniumtrichlorid $RuCl_3.3H_2O$;
- Rutheniumtrichlorid $RuCl_3.3H_2O$ in Verbindung mit mindestens einem Stabilisator, ausgewählt aus der Gruppe bestehend aus Jod, den kovalenten Jodiden und den quartären Ammoniumhalogeniden;
- tris-(Tetracarbonylruthenium) $Ru_3(CO)_{12}$ in Verbindung mit mindestens einem Stabilisator, ausgewählt aus der Gruppe bestehend aus Jod, den kovalenten Jodiden und den quartären Ammoniumhalogeniden; und
- Rutheniumjodid $RuJ_3$, bis-(Dichlortricarbonylruthenium) $[RuCl_2(CO_3)]_2$ und die Verbindungen $Ru(NH_3)_6Cl_3$ und $[Ru(NH_3)_5Cl]Cl_2$, gegebenenfalls weiters enthaltend mindestens einen Stabilisator, der ausgewählt ist aus der Gruppe bestehend aus Jod, den kovalenten Jodiden und den quartären Ammoniumhalogeniden.

## Claims

1. Process for the synthesis of saturated carboxylic esters from alkyl formate and an olefin, without the presence of substantial amounts of carbon monoxide, at a temperature of 120° to 280°C, under a pressure of 1 to 3000 bars, in the presence of a catalyst based on ruthenium coordinated by ligands selected from carbon monoxide, halogen atoms and amines, and in the presence of an amide as a solvent.

2. Process according to Claim 1 for the synthesis of methyl propionate, characterised in that the alkyl formate is methyl formate and in that the olefin is ethylene.

3. Process according to one of Claims 1 and 2, characterised in that the mole ratio of alkyl formate to ruthenium is between 500 and 10,000.

4. Process according to one of Claims 1 to 3, characterised in that it is carried out in the presence, in addition, of iodine, of a covalent iodide or of a quaternary ammonium halide.

5. Process according to Claim 4, characterised in that the mole ratio of iodine or of the said covalent iodide or quaternary ammonium halide relative to ruthenium is between 0.5 and 6.

6. Process according to one of Claims 1 to 5, characterised in that the solvent is dimethylformamide.

7. Process according to one of Claims 1 to 6, characterised in that the amide is used in the proportion of 0.5 to 3 volumes per volume of alkyl formate.

8. Process according to one of Claims 1 to 7, characterised in that the reaction between the alkyl formate and the olefin is performed for a time between 2 minutes and 10 hours.

9. Process according to one of Claims 1 to 8, characterised in that the alkyl formate and the olefin are in a substantially equimolar ratio.

10. Process according to one of Claims 1 to 9, characterised in that the catalyst is selected from:
- catalysts consisting essentially of ruthenium trichloride $RuCl_3.3H_2O$;
- catalysts consisting essentially of the combination of a ruthenium trichloride $RuCl_3.3H_2O$ together with at least one stabiliser selected from iodine, covalent iodides and quaternary ammonium halides;
- catalysts comprising tris(tetracarbonylruthenium) $Ru_3(CO)_{12}$ together with at least one stabiliser selected from iodine, covalent iodides and quaternary ammonium halides; and
- catalysts selected from ruthenium iodide $RuI_3$, bis(dichlorotricarbonylruthenium) $[RuCl_2(CO)_3]_2$ and the compounds $Ru(NH_3)_6Cl_3$ and $[Ru(NH_3)_5Cl]Cl_2$, comprising, where appropriate, in addition, at least one stabiliser selected from iodine, covalent iodides and quaternary ammonium halides.

11. Use as a catalyst for the synthesis of saturated carboxylic esters from alkyl formate and an olefin of at least one of:
- ruthenium trichloride $RuCl_3.3H_2O$;
- ruthenium trichloride $RuCl_3.3H_2O$ together with at least one stabiliser selected from iodine, covalent iodides and quaternary ammonium halides;
- tris(tetracarbonylruthenium) $Ru_3(CO)_{12}$ together with at least one stabiliser selected from iodine, covalent iodides and quaternary ammonium halides; and
- ruthenium iodide $RuI_3$, bis(dichlorotricarbonylruthenium) $[RuCl_2(CO)_3]_2$ and the compounds $Ru(NH_3)_6Cl_3$ and $[Ru(NH_3)_5Cl]Cl_2$, comprising, where appropriate, in addition, at least one stabiliser selected from iodine, covalent iodides and quaternary ammonium halides.